# FASCICULE DE BREVET EUROPEEN

(11) **EP 1 552 819 B1**
(45) Date de publication et mention de la délivrance du brevet: **21.08.2013**
(21) Numéro de dépôt: 04300881.2
(22) Date de dépôt: 13.12.2004
(51) Int. Cl.: C08J 3/12, C08J 9/24, C09D 101/26, C09D 101/28, C09D 103/00, C09D 105/00, C09D 105/04, C09D 105/12, C09D 105/14, A61K 9/28

(54) **Procédé de préparation d'une composition filmogène colorée.**
Verfahren zur Herstellung einer gefärbten filmbildenden Zusammensetzung.
Process for preparing a coloured film-forming composition.

(30) Priorité: 08.01.2004 FR 0450049
(43) Date de publication de la demande: 13.07.2005
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Trouve, Gérard, Castres, 81100 (FR); Girard, David, 81100, Castres (FR); Malandain, Michel, 78112, Fourqueux (FR)
(74) Mandataire: Conan, Philippe Claude

(56) Documents cités:
- EP-A- 0 207 384
- WO-A-89/04761
- FR-A- 2 548 675
- FR-A- 2 660 317
- US-A- 3 396 034
- DATABASE CHEMABS CHEMICAL ABSTRACTS SERVICE, COLUMBUS, OHIO, US; "Ready to use edible film coating" XP002292200 extrait de STN Database accession no. 125:257205 & IN 173 740 A (PRATAPRAI OZA KAMALESH DR) 2 juillet 1994 (1994-07-02)

## Description

La présente invention a pour objet un procédé de préparation de compositions filmogènes destinées à l'enrobage coloré de formes solides ingérables et plus particulièrement des comprimés pharmaceutiques, des confiseries ou des dragées, leur procédé de préparation, ainsi qu'un procédé d'enrobage et de coloration desdites formes.

Le pelliculage de comprimés par des films de polymères colorés est couramment utilisé dans l'industrie pharmaceutique. Ce procédé comprend la préparation d'une dispersion, de préférence aqueuse, contenant un polymère filmogène, un système colorant, de préférence des pigments ou des laques, et éventuellement un plastifiant, des charges et/ou des additifs technologiques, puis sa pulvérisation sur les comprimés en mouvement dans une turbine rotative perforée ou dans un lit fluidisé. Un courant d'air chaud, entrant à une température généralement supérieure ou égale à 40°C, assure le séchage de la dispersion pulvérisée et provoque la coalescence du film autour des comprimés. La couche déposée a une épaisseur de quelques dizaines de microns ; elle est opaque et colorée de manière homogène.

Pour rester à un niveau de viscosité acceptable, généralement inférieur à 1000 mPas, compatible avec un passage à travers des buses de pulvérisation, la dispersion utilisée est peu concentrée ; elle ne contient le plus souvent que de 10 à 20 % en poids de matière sèche.

Les composants utilisés pour réaliser ces films doivent être conformes aux réglementations en vigueur dans les domaines pharmaceutique, diététique ou alimentaire. C'est pourquoi, les polymères sont en général choisis parmi les dérivés de cellulose comme les hydroxypropyméthyl celluloses (HPMC), les méthyl celluloses (MC), les dérivés d'amidon comme par exemple, dextrines ou les maltodextrines filmogènes, les alcools polyvinyliques ou encore les polymères acryliques. Les plastifiants les plus utilisés dans le domaine pharmaceutique sont les polyéthylènes glycols (PEG) de poids moléculaires compris entre environ 300 et environ 8000. Par contre, ils ne sont pas autorisés pour des applications alimentaires pour lesquelles on utilise souvent la glycérine comme exemple de plastifiants. Comme charge, il y a la cellulose, le lactose, le sorbitol, le mannitol, le xylitol, la silice ou le talc. Parmi les additifs technologiques, il y a par exemple les tensioactifs tels que les polysorbates qui jouent le rôle de mouillants et de dispersants.

On peut avantageusement aussi utiliser des compositions prêtes à l'emploi contenant les mélanges de ces différents constituants sous forme de poudres, de granulés ou d'extrudâts. Les compositions prêtes à l'emploi présentent plusieurs avantages :
- La manipulation, le stockage, le contrôle d'un seul et unique produit ;
- Une meilleure reproductibilité des couleurs et des performances ;
- Une mise en dispersion plus aisée.

Ce dernier point est particulièrement important, car la dispersion de poudres introduites une à une dans un solvant aqueux est souvent une opération délicate à conduire en raison de la formation de mousse, de grumeaux ou d'agglomérats parfois qualifiés d'« yeux de poissons », qui sont très difficiles à disperser quand ils sont formés. De plus une dispersion complète nécessite souvent un temps d'agitation souvent très long.

La composition de revêtement comestible sèche sous forme de poudres décrite dans la demande de brevet français publiée sous le numéro FR 2 470 598, ne résout pas complètement ce problème car on observe lors de sa mise en oeuvre des phénomènes de ségrégation qui conduisent à des compositions hétérogènes dans les conteneurs de produits.

Les compositions plus stables sous formes de granulés ou d'extrudâts décrites dans les demandes de brevet publiées sous les numéros FR 2 548 675, FR 2 660 317 et WO 99/24020, ne présentent pas cet inconvénient car ce sont des agglomérats de plusieurs dizaines à plusieurs milliers de particules de matière, initialement individualisées, pouvant être de nature identique ou différente, qui ont été assemblées au moyen d'un liant liquide par un procédé de granulation humide dans un mélangeur - granulateur ou dans un lit fluidisé ou par un procédé d'extrusion, procédés décrits par exemple dans l'encyclopédie Kirk Othmer (3ème édition volume 17, page 281). On observe que les dimensions des granulés ou des extrudâts obtenus sont de l'ordre de 0,1 à 2 mm, alors que les particules de poudre initiales ont des dimensions de l'ordre de quelques dizaines de microns. De plus ces granulés ou ces extrudâts sont particulièrement faciles à manipuler car ils s'écoulent librement et n'induisent pas de poussières lors de leur mise en oeuvre. Cependant de telles formes prêtes à l'emploi sont assez difficiles et onéreuses à fabriquer. En effet le procédé de fabrication, tel que décrit par exemple dans les brevets publiés sous les numéros FR 2 548 675 et FR 2 660 317 comprend les étapes successives suivantes :
- le mouillage des poudres et pigments par une solution liante, afin d'obtenir une masse humide contenant de 30 à 60% d'eau,
- le séchage dans une étuve ou dans un lit fluidisé, et
- éventuellement le calibrage.

De plus ce procédé induit des opérations de nettoyage des nombreux appareils mis en jeu au cours des différentes étapes, qui sont particulièrement difficiles lorsque l'on prépare des compositions colorées.

Enfin, les compositions prêtes à l'emploi sous formes de poudres sèches, de granulés ou d'extrudâts se dispersent facilement dans l'eau, à condition de les verser progressivement sous agitation forte et ayant un fort effet de cisaillement, au moyen par exemple d'une pale défloculeuse. Elles permettent de préparer la dispersion aqueuse à pulvériser sur les comprimés, en un temps plus court que lorsque l'on introduit un à un tous les ingrédients nécessaires au pelliculage, mais cependant encore relativement long car il est de l'ordre de 30 à 45 minutes.

C'est pourquoi les inventeurs ont cherché à développer de nouveaux granulés et leur utilisation dans un procédé de préparation simple de compositions filmogènes prêtes à l'emploi, sous forme de granulés, stables, ne présentant pas de phénomènes de ségrégation au stockage, et se dispersant rapidement dans l'eau.

Selon un premier aspect, l'invention a pour objet le procédé tel que défini à la revendication 1.

Par masse volumique apparente µ, on désigne le rapport M/V dans lequel M représente la masse du matériau et V son volume apparent. La masse volumique apparente µ est déterminée selon le protocole expérimental 2- 9 -15 de la Pharmacopée européenne. µ est de préférence supérieur ou égal à 0,2 g / cm³.

Par porosité π, on désigne le rapport du volume des vides d'un matériau sur son volume total. π est égal à 0 pour un produit dense et tend vers 1 lorsque le matériau devient de plus en plus poreux. Elle répond à l'égalité : π = (a - µ) / a, dans laquelle a représente la masse volumique vraie du matériau.

Par polymère filmogène, on désigne principalement les polymères comestibles choisis parmi les dérivés de cellulose comme par exemple, les hydroxypropyméthyl celluloses (HPMC), les éthyl celluloses (EC), les méthyl celluloses (MC), les carboxyméthyl celluloses, les hydroxypropyl celluloses (HPC) ou les acétates ou phtalates de cellulose, les carraghénanes, les alginates de sodium, de potassium ou d'ammonium, les dérivés amidons modifiés filmogènes comme par exemple les dextrines les maltodextrines, les gommes guar, adragante, arabique ou xanthane et pour les applications non alimentaires, les polyvinylpyrrolidones, les alcools polyvinyliques, les polymères acryliques. On peut aussi utiliser des polymères déjà commercialisés sous forme de granulés comme, par exemple, de l'HPMC granulée connue sous le nom de PHARMACOAT™ G. On peut encore utiliser des mélanges de polymères différents comme par exemple les mélanges HPMC / EC.

La composition obtenue directement par le procédé tel que défini précédemment comprend plus particulièrement de 50 % à 100 % massique de polymères filmogène et tout particulièrement de 50 % à 90 % massique de polymères filmogène.

Par plastifiant, on entend principalement soit les plastifiants solubles comme les polyols en général et, plus particulièrement le sorbitol, le mannitol, le xylitol, le glycérol, le saccharose, les polyéthylène glycols, le propylène glycol, soit les plastifiants peu hydrosolubles tels que ceux comprenant une chaîne aliphatique comprenant au moins 12 atomes de carbone, par exemple, l'acide stéarique, les sels de l'acide stéarique, comme le stéarate de magnésium, le stéarate d'aluminium, l'acide stéarique polyéthoxylé les monoglycérides d'acides gras, les diglycérides d'acides gras et leurs dérivés estérifiés par l'acide acétique, l'acide tartrique ou l'acide lactique, les esters d'acides gras et de propylène glycol, les esters d'acides gras et de sorbitol, les esters d'acides gras et de sorbitanne, les esters d'acides gras et de mannitol, les esters d'acides gras et de mannitanne ou encore certains sucroesters, les sucroglycérides ou les esters de polyglycérol, en particulier ceux caractérisés par un nombre HLB inférieur à 7.

La composition obtenue directement par le procédé tel que défini précédemment comprend plus particulièrement de 0 % à 20 % massique d'un plastifiant ou d'un mélange de plastifiants.

Par charge inerte, on désigne principalement la cellulose microcristalline, le lactose, le talc ou la silice.

La composition obtenue directement par le procédé tel que défini précédemment comprend plus particulièrement de 0 % à 50 % massique d'une ou plusieurs charges inertes et tout particulièrement de 10% à 50 % massique d'une ou plusieurs charges inertes.

La composition obtenue directement par le procédé tel que défini précédemment a une teneur résiduelle en eau inférieure à 10 % massique, de préférence inférieure à 6 % massique. Sa masse volumique apparente µ est plus particulièrement inférieure ou égale à 0,4 g/cm³.

La composition obtenue directement par le procédé tel que défini précédemment présente généralement une répartition granulométrique telle qu'au plus 25% de sa masse totale a une granulométrie inférieure à 500 µm, au plus 15 % de sa masse totale a une granulométrie supérieure à 2000 µm et un diamètre moyen d'environ 800 µm, de préférence compris entre 600 et 1000 µm. Dans une version préférée, la répartition granulométrique dans la composition est telle qu'au plus 20% de sa masse totale a une granulométrie inférieure à 500 µm et au plus 15 % de sa masse totale a une granulométrie supérieure à 1500 µm.

Dans le procédé tel que défini précédemment, le soufflage d'air chaud, est de préférence effectué à une température entre 70°C et 100°C.

Comme les montrent les exemples expérimentaux développés plus loin dans le présent exposé, le procédé tel que défini précédemment permet, contrairement aux procédé classique de granulation, d'obtenir une composition sous forme de granulés poreux de masse volumique apparente µ, inférieure à 0,5 g / cm³.

Les agents de coloration utilisés dans l'invention sont ceux cités dans les pharmacopées ou dans les listes d'additifs alimentaires, référencés en Europe sous les numéros E 100 à E 172, comme, par exemple, les oxydes de fer, les oxydes de titane, de zinc ou de magnésium, les colorants absorbés sur laques d'alumines ou encore certains colorants naturels comme le caramel, les caroténoïdes, la riboflavine ou la chlorophylle. On peut avantageusement aussi utiliser des colorants composites constitués d'un assemblage de silicates de potassium et d'aluminium (mica), de dioxyde de titane et de colorants, tels que ceux commercialisés par la société Merck sous le nom de Candurin™.

Selon un aspect particulier de la présente invention, la composition filmogène obtenue directement par le procédé tel que défini ci-dessus comprend, en outre de 1 % à 20 % massique d'un ou plusieurs additifs technologiques.

Par additifs technologiques, on désigne plus particulièrement les agents favorisant l'adhésion du film, des plastifiants alimentaires ou pharmaceutiques liquides à température ordinaire, les agents édulcorants, les agents de masquage de goût et/ou les d'aromatisants alimentaires ou pharmaceutiques.

Par mélange à sec on indique que l'étape de fixation du procédé objet de la présente invention, se fait sans ajout d'eau, ni de solvant, par simple mélange des réactifs de départ, c'est à dire granulés poreux incolores, agents de coloration et, éventuellement, additifs technologiques qui, eux-mêmes peuvent être sous forme solide ou sous forme d'une solution organique. Il est bien entendu que le procédé tel que défini ci-dessus ne comprend ni d'étape de mouillage par une solution aqueuse liante, ni d'étape de séchage contrairement au procédé décrit dans les demandes de brevet français FR 2 548 675 et FR 2 660 317.

De manière tout à fait inattendue, les pigments et autres additifs technologiques ainsi mis en oeuvre, sont fixés de façon stable dans les granulés poreux de la composition objet de la présente invention, et le granulé coloré ainsi obtenu se dissout beaucoup plus rapidement dans l'eau que ceux préparés par les procédés de granulation décrits dans les brevets précédents.

Pour préparer le granulé coloré, le granulé poreux incolore, appelé par la suite granulé base, est placé dans un mélangeur performant tel que, par exemple, un mélangeur DIOSNA™ ou LODIGUE™, dans lequel on introduit un à un ou ensemble les colorants, laques, pigments et éventuellement, un ou plusieurs additifs technologiques de la composition filmogène. Le mélange à sec est effectué pendant quelques minutes, temps suffisant pour que les colorants, laques ou pigments et autres composants se fixent dans les pores et à la surface du granulé initial.

Le granulé coloré fmal obtenu se présente sous forme d'agglomérats plus ou moins sphériques, de granulométrie caractérisée par un pourcentage massique passant sur tamis de 400 µm inférieur à 25 %, une taille maximale de 2000 µm et un diamètre moyen d'environ 600 µm, de préférence compris entre 400 et 800 µm. Dans une version encore préférée de l'invention, le granulé coloré final présente une fraction massique passant à travers un tamis de 250 µm inférieure à 10 % et une fraction massique comprise entre 1000 µm et 2000 µm inférieure à 20 %. Sa densité est généralement supérieure ou égale à 0,2 et inférieure à 0,5, et de préférence supérieure ou égale à 0,3. Son humidité résiduelle est inférieure à 6 %. 100 g de granulés s'écoulent librement en moins de 20 secondes, selon le test 2-9-16 décrit dans la pharmacopée européenne 4^{ème} édition.

Sa dispersion dans l'eau est évaluée de manière standard dans un récipient en plastique de 2 litres, de diamètre 15 cm, dans lequel on pèse 880 g d'eau à température ambiante. On démarre une agitation au moyen d'une pale défloculeuse de diamètre 8 cm tournant à environ 500 tours par minute. Puis on introduit 120 g de granulé coloré progressivement pendant environ 1 minute. L'avancement de la dispersion est apprécié en mesurant périodiquement sur des prélèvements la quantité de solide restant sur un tamis de 125µm. La dispersion est qualifiée de rapide lorsqu'il ne reste plus aucun résidu sur le tamis de 125 µm au bout de 15 minutes.

Selon un aspect particulièrement intéressant de l'invention, la composition colorée telle que définie précédemment comprend au moins 20 % massique d'agents de coloration.

De telles compositions permettent la réalisation de pellicules colorées et homogènes sur les comprimés, dragées ou confiserie avec de très faibles dépôts secs, donc des opérations de pelliculage rapides et économiquement avantageuses. L'exemple 2 suivant décrit une telle application, qui permet de réaliser un pelliculage en environ 25 minutes alors que les compositions d'enrobage classiques, nécessitent des temps de mise en dispersion de l'ordre de 30 à 45 minutes puis des temps de pelliculage de d'encore 30 à 45 minutes supplémentaires.

Pour obtenir une dispersion pulvérisable, c'est à dire ayant une viscosité inférieure à 1000 mPas à 25°C, de préférence inférieure à 600 mPas, la teneur en matière sèche de la dispersion aqueuse est généralement comprise entre 7 % et 60 % massique, de préférence entre 10 % et 30 % massique.

C'est pourquoi l'invention a aussi pour objet une composition aqueuse de coloration caractérisée en ce qu'elle comprend :
- de 7% à 60 % d'une composition telle que définie précédemment et de 40 % à 93 % d'eau,
ainsi que le procédé de préparation de la composition aqueuse de coloration telle que définie ci-dessus, caractérisé en ce qu'il comprend le mélange dans l'eau d'une ou plusieurs compositions colorées telle que définie ci-dessus.

La teneur en matière sèche de la dispersion aqueuse est ajustée en fonction des polymères utilisés.

L'invention a enfin pour objet un procédé de pelliculage de comprimés, de dragées ou de confiseries, caractérisé en ce qu'il met en oeuvre la composition aqueuse de coloration telle que définie précédemment.

La dispersion aqueuse obtenue est pulvérisée sur les comprimés en mouvement dans une turbine rotative perforée ou dans un lit fluidisé. Un courant d'air chaud, entrant à une température généralement supérieure ou égale à 40°C, assure le séchage de la dispersion pulvérisée. Dans le cas de dragées ou confiseries, le pelliculage doit être effectué avec un air de séchage ayant une température de préférence comprise entre environ 30°C et 40°C.

Les exemples ci dessous illustrent l'invention et sont donnés à titre non limitatif.

### Exemple A: Préparation d'un granulé « base » en granulation humide (exemple comparatif)

On verse 16,8 kg d'HPMC 6 cps (PHARMACOAT™ 606) et 3,1 kg de cellulose microcristalline (VIVAPUR™ 105) dans un mélangeur - granulateur DIONA™ V-100. On homogénéise le mélange pendant 5 minutes puis on ajoute progressivement 55 litres d'eau à température ambiante pour obtenir une masse humide qui est ensuite broyée et séchée dans un lit fluidisé jusqu'à obtenir une humidité résiduelle de 1% environ.

Les granulés secs obtenus sont de couleur blanche ;

Leur masse volumique apparente, déterminée selon le protocole expérimental 2-9-15 de la Pharmacopée européenne, est égale à 0,66.

La répartition granulométrique, obtenue par tamisage de 100 g de granulés dans une colonne de tamis normalisés posés sur un tamiseur rotatif « LABOMODERNE™ S + S» vibrant à la vitesse 100 pendant 10 minutes, est la suivante :
9 % massique ont une taille < 315µm
37 % massique ont une taille dans l'intervalle [315 µm, 1000µm[, et
54 % massique ont une taille dans l'intervalle [1000 µm, 2000 µm[.

Leur temps d'écoulement, déterminé par le test 2-9-16 décrit dans la Pharmacopée européenne 4^{ème} édition, est inférieur à 10 secondes.

### Exemple 1: Préparation de granulés non colorés

On granule 12,6 kg d'HPMC 6 cps (PHARMACOAT™ 606) et 2,4 kg de cellulose microcristalline (VIVAPUR™ 105) dans un lit fluidisé GLAT™ GPCG 60. On homogénéise tout d'abord le mélange des deux poudres par soufflage d'air pendant 6 minutes puis on pulvérise progressivement 14 litres d'eau pour obtenir une masse humide, qui est ensuite séchée par soufflage d'air chaud à 95°C jusqu'à atteindre une humidité résiduelle de 4% environ.

Les granulés secs obtenus sont de couleur blanche;

Leur masse volumique apparente, déterminée selon le protocole expérimental 2-9-15 de la Pharmacopée européenne, est égale à 0,22.

La répartition granulométrie, obtenue par la méthode décrite dans l'exemple A, est la suivante :
6 % massique ont une taille < 400 µm
10 % massique dans l'intervalle [400 µm, 500 µm[,
58 % massique dans l'intervalle [500 µm, 1000 µm[,
26 % massique dans l'intervalle [1000 µm, 2000 µm[.

Son temps d'écoulement, déterminé par le test 2 -9 -16 décrit dans la Pharmacopée européenne 4^{ème} édition, est égal à 21 secondes.

### Exemple 2 : Composition granulée filmogène verte (Exemple selon l'invention)préparation et utilisation

### (a) - Préparation

On utilise les granulés non colorés préparés à l'exemple 1, pour fabriquer une composition filmogène verte en une seule étape, par simple mélange à sec.

Pour ce faire, on verse, dans un mélangeur DIOSNA™ V10, 1400 g de granulés non colorés, 534 g de pigment blanc (dioxyde de titane, colorant E 171), 24 g de jaune de quinoléine sous forme de laque d'alumine (colorant E 104) et 42 g de bleu patenté V sous forme de laque d'alumine (colorant E 131)

Après mélange des ces composés pendant 1 minute à vitesse 1, on obtient des granulés verts.

Leur masse volumique apparente, déterminée selon le protocole expérimental 2-9-15 de la Pharmacopée européenne, est égale à 0,39.

La répartition granulométrique, obtenue par la méthode décrite dans l'exemple A, est la suivante :
9 % massique < 250 µm,
15% massique dans l'intervalle [250 µm, 400 µm[,
13 % massique dans l'intervalle [400 µm, 500 µm[,
48 % massique dans l'intervalle [500 µm, 1000 µm[ et
15 % massique dans l'intervalle [1000 µm, 2000 µm[.

La réelle fixation des laques et pigments sur les granulés non colorés, est mise en évidence grâce aux résultats suivants :
La répartition granulométrique de la composition filmogène granulée verte finale est voisine de celle de la composition non colorée initiale (voir exemple 1). En effet, les agents de coloration sont de très fines particules solides de diamètre inférieurs à 100 µm. Si elles n'étaient pas intimement incorporées dans le granulé poreux incolore, la répartition granulométrique du produit coloré obtenu présenterait un taux de particules inférieure à 250 µm qui serait de l'ordre de 36 %.

Chacune des fractions granulométriques obtenues est remise en dispersion aqueuse contenant 15 % de matière sèche et les coordonnées tri chromatiques L, a, b, de chaque dispersion sont mesurées au moyen d'un chromamètre MINOLTA™ CR 200. Le tableau suivant montre qu'il n'y a pas de différence significative de couleur entre les différentes fractions, démontrant ainsi que les pigments se sont bien fixés de manière homogène sur l'ensemble des particules de granulé base.

| fraction | < 400 µm | [400-500 µm[ | [500, 1000 µm[ | [1000, 2000 µm[ |
|---|---|---|---|---|
| L | 68,0 | 68,6 | 67,7 | 67,5 |
| a | -27,3 | -28,1 | -28,0 | -27,6 |
| b | -6,2 | -6,8 | -7,0 | -6,8 |

Un fût contenant 5 kg de la composition filmogène granulée verte obtenue est soumis à des vibrations pendant plusieurs jours de façon à provoquer une éventuelle ségrégation des particules de pigments fines et denses. Des échantillons sont alors prélevés en haut, au milieu et en bas du fût et contrôlés pour en déterminer le pourcentage de fines (pourcentage massique de poudres passant à travers un tamis de 150 µm), pour en apprécier visuellement la couleur et pour déterminer les coordonnées trichromatiques d'une dispersion aqueuse à 15 % massique de matière sèche. Les résultats consignés dans le tableau suivant, ne font apparaître aucune différence significative entre les différents prélèvements, ce qui prouve l'homogénéité du contenu du fût et donc la réalité de la fixation des laques sur les granulés non colorés.

| | Avant vibrations | Après vibrations | | |
|---|---|---|---|---|
| | | Prélèvement en haut du fût | Prélèvement au milieu du fût | Prélèvement en bas du fût |
| Fines (% massique) | 6,5 % | 5,1 % | 6,9 % | 6,1 % |
| Couleur pantone | 3252C | 3252C | 3252C | 3252C |
| L | 68,0 | 68,2 | 68,5 | 67,7 |
| a | -27,5 | -27,0 | -27,4 | -28,0 |
| b | -6,8 | -6,1 | -6,9 | -7,0 |

### (b) - Enrobage de comprimés

### (i) - préparation de la dispersion aqueuse

75 g de la composition filmogène granulée verte préparée au paragraphe précédent, sont dispersés dans 425 g d'eau à température ambiante au moyen d'une pale défloculeuse tournant à 500 tours par minutes. Après 15 minutes, la totalité des granulés verts sont dispersés dans l'eau et la dispersion est utilisée pour pelliculer des comprimés pharmaceutiques de 550 mg et de diamètre 12 mm.

A titre de comparaison, on a préparé une dispersion aqueuse de granulés de même composition chimique que ceux préparés au paragraphe (a) précédent, mais qui ont été obtenus par granulation humide selon le procédé décrit dans le brevet français publié sous le numéro FR 2 548 675. Les résultats consignés dans le tableau suivant, font apparaître que la dispersion aqueuse de granulés selon l'invention ne contient plus aucun résidu solide après quinze minutes d'agitation, alors que celle de l'état de la technique en contient encore après plus de trente minutes d'agitation.

**Tableau 1 : présence de résidus solides sur filtre de 125 µm dans les dispersions aqueuses de granulés soumis à agitation d'une pale défloculeuse**

| Temps ( minutes ) | 5 | 15 | 30 | 45 |
|---|---|---|---|---|
| Granulé selon l'invention | OUI | AUCUN | AUCUN | AUCUN |
| Granulé selon FR 2 548 675. | OUI | OUI | FAIBLE | AUCUN |

### (ii) - Enrobage de comprimés

La dispersion obtenue au paragraphe (i) précédent, est pulvérisée sur 1000 g de comprimés placés dans une turbine à dragéifier ERWEKA™, au moyen d'un pistolet BINKS™, muni d'une buse de pulvérisation de 0,8 mm de diamètre. La pulvérisation en fines gouttelettes est assurée par de l'air comprimé à 2.10⁵ Pa (2 bars). Le débit de pulvérisation est maintenu à 6 g/minute, la température d'air de séchage est de 55°C et la température des comprimés maintenue voisine de 30°C.

Des prélèvements sont effectués après dépôt de 0,5 %, 0,75 %, 1 %, 1,25 %, et 1,5 % massique de matière sèche sur les comprimés. On constate des défauts d'uniformité de couleur et de couvrant jusqu'à un taux de coloration représentant le dépôt de 0,5 % de dépôt sec. Dès 0,75 % de dépôt, les comprimés sont de couleur verte très uniforme.

Le temps nécessaire à la réalisation d'un pelliculage homogène est donc réduit à 8 minutes plus les 15 minutes de temps de préparation de la dispersion ce qui représente un gain d'au moins 50% de temps par rapport au pelliculage réalisé dans les mêmes conditions avec la dispersion de l'état de la technique.

### Exemple 3 : Préparation d'une composition filmogène rouge (Exemple selon l'invention)

On utilise la composition non colorée préparée à l'exemple 1, pour fabriquer une composition filmogène rouge en une seule étape par simple mélange à sec. On verse dans un mélangeur DIOSNA™ V 10, 1400 g de granulés non colorés, 96 g de pigment blanc (dioxyde de titane E 171), 102 g de tartrazine sous forme de laque d'alumine (E 102), 174 g de jaune orangé sous forme de laque d'alumine (E 110) et 228 g d'érythrosine sous forme de laque d'alumine (E 127). Après mélange de ces composés pendant 1 minute à vitesse 1, on obtient des granulés rouges.

Leur masse volumique apparente, déterminée selon le protocole expérimental 2-9-15 de la Pharmacopée européenne, est égale à 0,40 ;

La répartition granulométrie, obtenue par la méthode décrite dans l'exemple A, est la suivante :
6 % massique < 250 µm
14 % dans l'intervalle [250 µm, 400 µm[,
12 % dans l'intervalle [400 µm, 500µm[,
52 % dans l'intervalle [500 µm, 1000 µm[ et
16 % dans l'intervalle [1000 µm, 2000 µm[

Cette répartition granulométrique est voisine de celle des granulés non colorés initiaux. ce qui prouve la réelle fixation des laques et pigments sur le granulé base.

Une dispersion à 15 % dans l'eau de cette composition filmogène granulée rouge est ensuite préparée avec d'une pale défloculeuse tournant à 500 tours par minute. La dissolution est complète au bout de 15 minutes.

### Exemple 4 : Fabrication d'une composition filmogène plastifiée colorée préparation et utilisation (exemple selon l'invention)

On utilise la composition non colorée préparée à l'exemple 1, pour fabriquer une composition filmogène rouge en une seule étape par simple mélange à sec.

On verse, dans un mélangeur DIOSNA™ V 10, 1000 g de granulés non colorés, auquel on ajoute progressivement, au moyen d'un pistolet BINKS™, 135 g de polyéthylène glycol 400 en tant que plastifiant liquide. Le mélange est réalisé sous agitation à faible vitesse pour bien répartir le plastifiant sans endommager le granulé non coloré. On ajoute ensuite successivement 135 g de dioxyde de titane (E 171) et 260 g de laque d'érythrosine (E 127). Après mélange de ces composés pendant 1,5 minutes à vitesse 1, on obtient des granulés rouges qui s'écoulent librement, de granulométrie et de masse volumique apparente (µ = 0,40) très voisines de celles données dans l'exemple 3.

Une dispersion dans l'eau à 12 % de ce granulé est préparée sous agitation au moyen d'une pale défloculeuse en 15 minutes environ. Elle est pulvérisée sur 3000 g de comprimés placés dans une turbine ventilée DRIACOATER™, au moyen d'un pistolet BINKS™ muni d'une buse de pulvérisation de 0,8 mm. La pulvérisation en fines gouttelettes est assurée par de l'air comprimé à 2.10⁵ Pa (2 bars). Le débit de pulvérisation est maintenu à 10 g / minute. La température d'air de séchage entrant est de 65 °C, celle de l'air sortant est de 40 °C, et celle des comprimés est ainsi maintenue à 33 °C. Après dépôt de 3 % de matière sèche, on obtient des comprimés pelliculés de couleur rouge très uniforme.

A titre de comparaison, on a réalisé dans les mêmes conditions opératoires, une dispersion de SEPIFILM™ 5938, produit de composition chimique identique mais préparé par granulation humide selon le mode opératoire décrit dans le brevet publié sous le numéro FR 2 548 675. Le temps de mise en dispersion nécessaire pour qu'aucune particule de diamètre supérieur à 125 µm ne subsiste est de 40 minutes.

Le pelliculage de comprimés au moyen de cette dispersion de SEPIFILM™ 5938 conduit au même résultat que celui obtenu avec la composition selon l'invention, mais la préparation de la dispersion est nettement plus lente qu'avec la composition rouge selon l'invention.

### Exemple 5 : Composition filmogène pour enrobage de confiseries ; préparation et utilisation (exemple selon l'invention)

### (a) - Préparation

### (i) - Composition non colorée

On prépare une composition non colorée par le procédé décrit à l'exemple 1 ci-dessus à partir des constituants suivants :

| | |
|---|---|
| HPMC : | 75 % massique |
| VIVAPUR™ 105 : | 11 % massique |
| Acide stéarique : | 14 % massique |

La masse volumique apparente, déterminée selon le protocole expérimental 2-9-15 de la Pharmacopée européenne, est égale à 0,23 ;

La répartition granulométrie, obtenue par la méthode décrite dans l'exemple A, est la suivante :
2 % massique < 400 µm
10 % dans l'intervalle [400 µm, 500 µm[,
78 % dans l'intervalle [500 µm, 1000µm[, et
10 % dans l'intervalle [1000 µm, 2000 µm[

### (ii) - composition colorée

On verse dans un mélangeur DIOSNA™ V 10, 1400 g des granulés non colorés préparés au paragraphe précédent, 480 g de dioxyde de titane (E 171), 22 g de jaune de quinoléine sous forme de laque d'alumine (E 104), 38 g de bleu patenté V sous forme de laque d'alumine (E 131) et 60 g de mica titane (CANDURIN™ Silver fine). Après mélange des composés pendant 1 minute à vitesse 1, on obtient un granulé vert. qui s'écoule librement.

La masse volumique apparente, déterminée selon le protocole expérimental 2- 9 - 15 de la Pharmacopée européenne, est égale à 0,37 ;

La répartition granulométrie obtenue est voisine de celle de la composition non colorée initiale.

### (b) - Enrobage de confiserie

On prépare une dispersion de 75 g de granulés verts, obtenus au paragraphe précédent, dans 425 g d'eau à température ambiante au moyen d'une pale défloculeuse. La dissolution est appréciée en mesurant périodiquement sur des prélèvements la quantité de solide restant sur un tamis de 125 µm. Au bout de 15 minutes, la totalité des granulés verts est dispersé dans l'eau et la dispersion résultante est utilisée pour pelliculer 3000 g de gommes à mâcher, dans une turbine ventilée DRIACOATER™ par pulvérisation au moyen d'un pistolet BINKS™, muni d'une buse de pulvérisation de 0,8 mm de diamètre.

La pulvérisation en fines gouttelettes est assurée par de l'air comprimé à 2.10⁵ Pa (2 bars). Le débit de pulvérisation est augmenté progressivement en une heure, de 1,2 g à 4 g de dispersion par minute. La température de l'air entrant est maintenue autour de 32°C. Au bout de 1 heure 40 minutes, 315 g de dispersion ont été pulvérisés, ce qui correspond à un dépôt sec de 1,5 % sur les gommes à mâcher. On obtient des gommes à mâcher colorés en vert, très brillants, de couleur homogène et sans marbrures.

### Exemple 6 : Composition filmogène brune pour enrobage de comprimés préparation et utilisation (exemple selon l'invention)

### (a) - Préparation

### (i) - composition non colorée

On prépare une composition non colorée dans un lit d'air fluidisé industriel Glatt GPCG300 en granulant la composition ci dessous :

| | |
|---|---|
| HPMC : | 168 kg |
| Cellulose microcristalline : | 31 kg |

au moyen de 337 kg d'eau introduite à un débit de l'ordre de 2kg/min. Durant l'opération de granulation, la température d'air entrant est maintenue à 85°C.

On obtient en final un granulé poreux présentant une humidité résiduelle de 2% et dont la masse volumique apparente, déterminée selon le protocole expérimental 2-9-15 de la Pharmacopée européenne, est égale à 0,25 ;

La répartition granulométrie, obtenue par la méthode décrite dans l'exemple A, est la suivante :
0,3 % massique < 400 µm
6,9 % dans l'intervalle [400 µm, 500 µm[,
72,8 % dans l'intervalle [500 µm, 1000µm[, et
11,4 % dans l'intervalle [1000 µm, 2000 µm[

### (ii) - composition colorée

On verse dans un mélangeur DIOSNA™ V 100, 14000 g des granulés non colorés préparés au paragraphe précédent, 5400 g de dioxyde de titane (E 171), 480g d'oxyde de fer jaune (E 171J), 120 g d'oxyde de fer rouge (E 171R). Après mélange sec des composés, on obtient un granulé brun qui s'écoule librement.

La masse volumique apparente, déterminée selon le protocole expérimental 2-9-15 de la Pharmacopée européenne, est égale à 0,38 ;

La répartition granulométrie, obtenue par la méthode décrite dans l'exemple A, est la suivante :
5,0 % massique < 400 µm
9,6 % dans l'intervalle [400 µm, 500 µm[,
76,8 % dans l'intervalle [500 µm, 1000µm[, et
8,6 % dans l'intervalle [1000 µm, 2000 µm[

Elle est très voisine de celle de la composition non colorée initiale, ce qui prouve à nouveau que les particules d'agent colorant ont bien été incorporées à l'intérieur des granulés poreux non colorés.

### (b) - Enrobage de comprimés

On prépare une dispersion de 75 g de granulés bruns, obtenus au paragraphe précédent, dans 425 g d'eau à température ambiante au moyen d'une pale défloculeuse. La dissolution est appréciée en mesurant périodiquement sur des prélèvements la quantité de solide restant sur un tamis de 125 µm. Au bout de 15 minutes, la totalité des granulés bruns est dispersée dans l'eau et la dispersion résultante est utilisée pour pelliculer 3000 g de comprimés, dans une turbine ventilée DRIACOATER™ par pulvérisation au moyen d'un pistolet BINKS™, muni d'une buse de pulvérisation de 0,8 mm de diamètre.

La pulvérisation en fines gouttelettes est assurée par de l'air comprimé à 2.105 Pa (2 bars). Le débit de pulvérisation est maintenu à 10 g/minute. La température d'air de séchage entrant est de 65°C, celle de l'air sortant est de 40°C, et celle des comprimés est ainsi maintenue à 33°C. Après dépôt de 3 % de matière sèche, on obtient des comprimés pelliculés de couleur brune très uniforme.

## Revendications

1. Procédé de préparation d'une composition filmogène colorée sous forme de granulés de porosité non nulle, de masse volumique apparente µ inférieure à 0,5 g/cm^{3,} comprenant pour 100 % massique de constituants :
de 30 % à 100 % massique d'un polymère filmogène ou d'un mélange de plusieurs de ces polymères,
de 0 % à 30 % massique d'un plastifiant ou d'un mélange de plastifiants,
de 0 % à 70 % massique d'une ou plusieurs charges inertes, avec un ou plusieurs agents de coloration et éventuellement un ou plusieurs additifs technologiques, **caractérisé en ce qu'**un polymère filmogène ou un mélange de plusieurs de ces polymères, éventuellement le plastifiant et/ou éventuellement la charge inerte sont homogénéisés dans un lit fluidisé par soufflage d'air, dans lequel on pulvérise ensuite et progressivement de l'eau jusqu'à former une masse humide qui est simultanément ou ensuite séchée par soufflage d'air chaud, pour obtenir une composition filmogène sous forme de granulés de porosité non nulle, de masse volumique apparente µ inférieure à 0,5 g/cm³, comprenant pour 100 % massique de constituants :
de 30 % à 100 % massique d'un polymère filmogène ou d'un mélange de plusieurs de ces polymères,
de 0 % à 30 % massique d'un plastifiant ou d'un mélange de plastifiants et,
de 0 % à 70 % massique d'une ou plusieurs charges inertes,
et **en ce que** la composition ainsi obtenue est mélangée à sec avec un ou plusieurs agents de coloration, la dite composition comprenant alors de 1% à 40% massique d'un ou plusieurs agents de coloration

2. Procédé tel que défini à la revendication 1, de préparation d'une composition dans laquelle les polymères filmogènes sont choisis parmi les hydroxypropyméthyl celluloses (HPMC), les éthyl celluloses (EC), les méthyl celluloses (MC), les carboxyméthyl celluloses, les hydroxypropyl celluloses (HPC) ou les acétates ou phtalates de cellulose, les carraghénanes, les alginates de sodium, de potassium ou d'ammonium, les amidons modifiés, dextrines, maltodextrines, les gommes guar, adragante, arabique ou xanthane, les polyvinylpyrrolidones, les alcools polyvinyliques ou les polymères acryliques.

3. Procédé tel que défini à l'une des revendications 1 ou 2, de préparation d'une composition comprenant de 50 % à 100 % massique et plus particulièrement de 50 % à 90 % massique d'un polymère filmogène ou d'un mélange de plusieurs de ces polymères.

4. Procédé tel que défini à l'une des revendications 1 à 3, de préparation d'une composition dans laquelle les plastifiants sont choisis parmi le sorbitol, le mannitol, le xylitol, le glycérol, le saccharose, les polyéthylène glycols, le propylène glycol, l'acide stéarique, les sels de l'acide stéarique, comme le stéarate de magnésium, le stéarate d'aluminium, l'acide stéarique polyéthoxylé les monoglycérides d'acides gras, les diglycérides d'acides gras et leurs dérivés estérifiés par l'acide acétique, l'acide tartrique ou l'acide lactique, les esters d'acides gras et de propylène glycol, les esters d'acides gras et de sorbitol, les esters d'acides gras et de sorbitanne, les esters d'acides gras et de mannitol, les esters d'acides gras et de mannitanne ou encore certains sucroesters, les sucroglycérides ou les esters de polyglycérol.

5. Procédé tel que défini à l'une des revendications 1 à 4, de préparation d'une composition comprenant de 0 % à 20 % massique d'un plastifiant ou d'un mélange de plastifiants.

6. Procédé tel que défini à l'une des revendications 1 à 5, de préparation d'une composition dans laquelle la charge inerte est choisie parmi la cellulose microcristalline, le lactose, le talc ou la silice.

7. Procédé tel que défini à l'une des revendications 1 à 6, de préparation d'une composition comprenant de 0 % à 50 % massique et plus particulièrement de 10 % à 50 % massique d'une ou plusieurs charges inertes.

8. Procédé tel que défini à l'une des revendications 1 à 7, de préparation d'une composition comprenant en outre jusqu'à 10 % massique d'eau et de préférence moins de 6 % massique d'eau.

9. Procédé tel que défini à l'une des revendications 1 à 8, de préparation d'une composition dont la masse volumique apparente µ est inférieure à 0,4 g / cm³.

10. Procédé tel que défini à l'une des revendications 1 à 9, de préparation d'une composition dont la répartition granulométrique est telle qu'au plus 25 % massique des granulés ont une granulométrie inférieure à 500µm, au plus 15 % massique des granulés ont une granulométrie supérieure à 2000 µm.

11. Procédé tel que défini à l'une des revendications 1 à 10, de préparation d'une composition dans laquelle les agents de coloration sont choisis parmi les oxydes de fer, les oxydes de titane, de zinc ou de magnésium, les colorants absorbés sur laques d'alumines ou encore colorants naturels comme le caramel, les caroténoïdes, la riboflavine ou la chlorophylle.

12. Procédé tel que défmi à l'une des revendications 1 à 11, de préparation d'une composition colorée comprenant en outre de 1 % à 20 % massique d'un ou plusieurs additifs technologiques choisis parmi les agents favorisant l'adhésion du film, des plastifiants alimentaires ou pharmaceutiques liquides à température ordinaire, les agents édulcorants, les agents de masquage de goût et/ou les d'aromatisants alimentaires ou pharmaceutiques.

13. Procédé tel que défini à l'une des revendications 1 à 12, de préparation d'une composition colorée comprenant au moins 20 % massique d'agents de coloration.

## Patentansprüche

1. Verfahren zur Herstellung einer farbigen filmbildenden Zusammensetzung in Form von Granulat mit einer Porosität, die nicht null ist, einer scheinbaren Dichte µ kleiner als 0,5 g/cm³, die für 100 Gew.-% die folgenden Bestandteile umfasst:
30 % bis 100 Gew.-% eines filmbildenden Polymers oder eines Gemischs aus mehreren dieser Polymere,
0 % bis 30 Gew.-% eines Weichmachers oder eines Gemischs aus Weichmachern,
0 Gew.-% bis 70 Gew.-% eines oder mehrerer inerter Füllstoffe, mit einem oder mehreren Färbemitteln und gegebenenfalls einem oder mehreren technischen Hilfsstoffen,
**dadurch gekennzeichnet, dass** ein filmbildendes Polymer oder ein Gemisch mehrerer dieser Polymere, gegebenenfalls der Weichmacher und/oder gegebenenfalls der inerte Füllstoff in einem Fließbett durch Einblasen von Luft homogenisiert werden, in dem anschließend und nach und nach Wasser zerstäubt wird, bis sich eine feuchte Masse bildet, die gleichzeitig oder anschließend durch Einblasen von Heißluft getrocknet wird, um eine filmbildende Zusammensetzung in Form von Granulat mit einer Porosität, die nicht null ist, einer scheinbaren Dichte µ kleiner als 0,5 g/cm³ zu erhalten, die für 100 Gew.-% die folgenden Bestandteile umfasst:
30 % bis 100 Gew.-% eines filmbildenden Polymers oder eines Gemischs aus mehreren dieser Polymere,
0 % bis 30 Gew.-% eines Weichmachers oder eines Gemischs aus Weichmachern, und
0 Gew.-% bis 70 Gew.-% eines oder mehrerer inerter Füllstoffe,
**dadurch gekennzeichnet, dass** die so erhaltene Zusammensetzung trocken mit einem oder mehreren Färbemitteln gemischt wird, wobei die Zusammensetzung dann 1 Gew.-% bis 40 Gew.-% eines oder mehrerer Färbemittel umfasst.

2. Verfahren wie in Anspruch 1 definiert, zur Herstellung einer Zusammensetzung, wobei die filmbildenden Polymere ausgewählt sind aus Hydroxypropymethylcellulosen (HPMC), Ethylcellulosen (EC), Methylcellulosen (MC), Carboxymethylcellulosen, Hydroxypropylcellulosen (HPC) oder Celluloseacetaten oder - phtalaten, Carragenanen, Natrium-, Kalium- oder Ammoniumalginaten, modifizierten Stärken, Dextrinen, Maltodextrinen, Guargummis, Tragantgummis, Gummi arabicum oder Xanthangummis, Polyvinylpyrrolidonen, Polyvinylalkoholen oder Acrylpolymeren.

3. Verfahren wie in einem der Ansprüche 1 oder 2 definiert, zur Herstellung einer Zusammensetzung, umfassend 50 Gew.-% bis 100 Gew.-% und insbesondere 50 Gew.-% bis 90 Gew.-% eines filmbildenden Polymers oder eines Gemischs aus mehreren dieser Polymere.

4. Verfahren wie in einem der Ansprüche 1 bis 3 definiert, zur Herstellung einer Zusammensetzung, wobei die Weichmacher ausgewählt sind aus Sorbitol, Mannitol, Xylitol, Glycerol, Saccharose, Polyethylenglycolen, Propylenglycol, Stearinsäure, Stearinsäuresalzen, wie Magnesiumstearat, Aluminiumstearat, polyethoxylierter Stearinsäure, Fettsäuremonoglyceriden, Fettsäurediglyceriden und ihren mit Essigsäure, Weinsäure oder Milchsäure veresterten Derivaten, Estern von Fettsäuren und
Propylenglycol, Estern von Fettsäuren und Sorbitol, Estern von Fettsäuren und Sorbitan, Estern von Fettsäuren und Mannitol, Estern von Fettsäuren und Mannitan oder auch bestimmten Zuckerestern, Zuckerglyceriden oder Polyglycerolestern.

5. Verfahren wie in einem der Ansprüche 1 bis 4 definiert, zur Herstellung einer Zusammensetzung, umfassend 0 Gew.-% bis 20 Gew.-% eines Weichmachers oder eines Gemischs aus Weichmachern.

6. Verfahren wie in einem der Ansprüche 1 bis 5 definiert, zur Herstellung einer Zusammensetzung, wobei der inerte Füllstoff aus mikrokristalliner Cellulose, Lactose, Talk oder Siliciumdioxid ausgewählt ist.

7. Verfahren wie in einem der Ansprüche 1 bis 6 definiert, zur Herstellung einer Zusammensetzung, umfassend 0 Gew.-% bis 50 Gew.-% und insbesondere 10 Gew.-% bis 50 Gew.-% eines oder mehrerer inerter Füllstoffe.

8. Verfahren wie in einem der Ansprüche 1 bis 7 definiert, zur Herstellung einer Zusammensetzung, ferner umfassend bis zu 10 Gew.-% Wasser und bevorzugt weniger als 6 Gew.-% Wasser.

9. Verfahren wie in einem der Ansprüche 1 bis 8 definiert, zur Herstellung einer Zusammensetzung, deren scheinbare Dichte µ kleiner als 0,4 g/cm³ ist.

10. Verfahren wie in einem der Ansprüche 1 bis 9 definiert, zur Herstellung einer Zusammensetzung, deren Korngrößenverteilung so ist, dass höchstens 25 Gew.-% des Granulats eine Granulometrie von weniger als 500 µm aufweisen, höchsten 15 Gew.-% des Granulats eine Granulometrie von mehr als 2.000 µm aufweisen.

11. Verfahren wie in einem der Ansprüche 1 bis 10 definiert, zur Herstellung einer Zusammensetzung, wobei die Färbemittel ausgewählt sind aus Eisenoxiden, Titan-, Zink- oder Magnesiumoxiden, Farbstoffen, die auf Aluminiumoxidlacken absorbiert sind oder auch natürlichen Farbstoffen, wie Caramel, Carotinoiden, Riboflavin oder Chlorophyll.

12. Verfahren wie in einem der Ansprüche 1 bis 11 definiert, zur Herstellung einer farbigen Zusammensetzung, ferner umfassend 1 Gew.-% bis 20 Gew.-% eines oder mehrerer technologischer Hilfsstoffe, ausgewählt aus Mitteln, die die Adhäsion des Films fördern, alimentären oder pharmazeutischen Weichmachern, die bei Umgebungstemperatur flüssig sind, Süßstoffen, Geschmacksmaskierungsmitteln und/oder alimentären oder pharmazeutischen Aromastoffen.

13. Verfahren wie in einem der Ansprüche 1 bis 12 definiert, zur Herstellung einer farbigen Zusammensetzung, umfassend mindestens 20 Gew.-% eines Färbemittels.

## Claims

1. Process for preparing a coloured film-forming composition in the form of granules having a non-zero porosity, having a bulk density µ less than 0.5 g/cm³, comprising, for 100% by weight of components:
from 30% to 100% by weight of a film-forming polymer or of a mixture of a plurality of these polymers,
from 0% to 30% by weight of a plasticiser or of a mixture of plasticisers,
from 0% to 70% by weight of one or more inert fillers, with one or more colouring agents and optionally one or more technological additives,
**characterised in that** a film-forming polymer or a mixture of a plurality of these polymers, optionally the plasticiser and/or optionally the inert filler are homogenised in a fluidised bed by blowing with air, into which water is then gradually sprayed until a wet mass is formed which is simultaneously or
subsequently dried by blowing with hot air, to obtain a film-forming composition in the form of granules having a non-zero porosity, having a bulk density µ less than 0.5 g/cm³, comprising for 100% by weight of components:
from 30% to 100% by weight of a film-forming polymer or of a mixture of a plurality of these polymers,
from 0% to 30% by weight of a plasticiser or of a mixture of plasticisers, and
from 0% to 70% by weight of one or more inert fillers,
and **in that** the composition thus obtained is dry mixed with one or more colouring agents, said composition then comprising from 1% to 40% by weight of one or more colouring agents.

2. Process according to claim 1, for preparing a composition in which the film-forming polymers are selected from hydroxypropylmethyl celluloses (HPMC), ethyl celluloses (EC), methyl celluloses (MC), carboxymethyl celluloses, hydroxypropyl celluloses (HPC) or cellulose acetates or phthalates, carrageenans, sodium, potassium or ammonium alginates, modified starches, dextrins, maltodextrins, guar gum, gum tragacanth, gum arabic or xanthan gum, polyvinylpyrrolidones, polyvinyl alcohols or acrylic polymers.

3. Process according to either claim 1 or claim 2, for preparing a composition comprising from 50% to 100% by weight and more particularly from 50% to 90% by weight of a film-forming polymer or of a mixture of a plurality of these polymers.

4. Process according to any of claims 1 to 3, for preparing a composition in which the plasticisers are selected from sorbitol, mannitol, xylitol, glycerol, sucrose, polyethylene glycols, propylene glycol, stearic acid, stearic acid salts, such as magnesium stearate or aluminium stearate, polyethoxylated stearic acid, fatty acid monoglycerides, fatty acid diglycerides and their derivatives esterified by acetic acid, tartaric acid or lactic acid, esters of fatty acids and of propylene glycol, esters of fatty acids and of sorbitol, esters of fatty acids and of sorbitan, esters of fatty acids and of mannitol, esters of fatty acids and of mannitan or certain sucrose esters, sucroglycerides or polyglycerol esters.

5. Process according to any of claims 1 to 4, for preparing a composition comprising from 0% to 20% by weight of a plasticiser or of a mixture of plasticisers.

6. Process according to any of claims 1 to 5, for preparing a composition in which the inert filler is selected from microcrystalline cellulose, lactose, talc or silica.

7. Process according to any of claims 1 to 6, for preparing a composition comprising from 0% to 50% by weight and more particularly from 10% to 50% by weight of one or more inert fillers.

8. Process according to any of claims 1 to 7, for preparing a composition further comprising up to 10% by weight of water and preferably less than 6% by weight of water.

9. Process according to any of claims 1 to 8, for preparing a composition of which the bulk density µ is less than 0.4 g/cm³.

10. Process according to any of claims 1 to 9, for preparing a composition of which the particle size distribution is such that at most 25% by weight of the granules have a particle size less than 500 µm and at most 15% by weight of the granules have a particle size greater than 2000 µm.

11. Process according to any of claims 1 to 10, for preparing a composition in which the colouring agents are selected from iron oxides, titanium, zinc or magnesium oxides, colorants absorbed on alumina lakes or natural colorants, such as caramel, carotenoids, riboflavin or chlorophyll.

12. Process according to any of claims 1 to 11, for preparing a coloured composition further comprising from 1% to 20% by weight of one or more technological additives selected from agents which promote adhesion of the film, food plasticisers or pharmaceutical plasticisers which are liquid at ambient temperature, sweeteners, agents for masking taste and/or food flavouring agents or pharmaceutical flavouring agents.

13. Process according to any of claims 1 to 12, for preparing a coloured composition comprising at least 20% by weight of colouring agents.
